(19) Europäisches Patentamt
European Patent Office
Office européen des brevets

(11) **EP 1 525 590 B1**

(12) **EUROPÄISCHE PATENTSCHRIFT**

(45) Veröffentlichungstag und Bekanntmachung des Hinweises auf die Patenterteilung:
**25.07.2007 Patentblatt 2007/30**

(21) Anmeldenummer: 03740434.0

(22) Anmeldetag: **08.07.2003**

(51) Int Cl.:
*G21F 1/12* *(2006.01)*

(86) Internationale Anmeldenummer:
**PCT/EP2003/007304**

(87) Internationale Veröffentlichungsnummer:
**WO 2004/013865 (12.02.2004 Gazette 2004/07)**

(54) **ABGESCHIRMTER RAUM FÜR DIE IONENTHERAPIE MIT ABSCHIRMWIRKUNG FÜR NEUTRONEN BIS IN DEN ENERGIEBEREICH GEV**

SCREENED CHAMBER FOR ION THERAPY WITH PROTECTION AGAINST NEUTRONS HAVING ENERGY UP TO GEV

CHAMBRE BLINDEE UTILISEE A DES FINS DE TRAITEMENT IONIQUE AVEC PROTECTION CONTRE LES NEUTRONS DONT LE DOMAINE D'ENERGIE PEUT ATTEINDRE LE GEV

(84) Benannte Vertragsstaaten:
**AT BE BG CH CY CZ DE DK EE ES FI FR GB GR HU IE IT LI LU MC NL PT RO SE SI SK TR**

(30) Priorität: **01.08.2002 DE 10235116**

(43) Veröffentlichungstag der Anmeldung:
**27.04.2005 Patentblatt 2005/17**

(73) Patentinhaber: **Gesellschaft für Schwerionenforschung mbH 64291 Darmstadt (DE)**

(72) Erfinder:
• **FEHRENBACHER, Georg 64367 Mühltal (DE)**
• **GUTERMUTH, Frank 64625 Bensheim (DE)**
• **RADON, Torsten 61239 Ober-Mörlen (DE)**

(56) Entgegenhaltungen:
EP-A- 0 986 070          DE-A- 19 517 415
US-A- 3 995 163          US-A- 5 703 918

• **S.AGOSTEO: "Radiation Protection at Medical Accelerators" RADIATION PROTECTION DOSIMETRY, Bd. 96, Nr. 4, 2001, Seiten 393-406, XP009016939 in der Anmeldung erwähnt**
• **BRAHME A ET AL: "Design of a centre for biologically optimised light ion therapy in Stockholm" NUCLEAR INSTRUMENTS & METHODS IN PHYSICS RESEARCH, SECTION - B: BEAM INTERACTIONS WITH MATERIALS AND ATOMS, NORTH-HOLLAND PUBLISHING COMPANY. AMSTERDAM, NL, Bd. 184, Nr. 4, Dezember 2001 (2001-12), Seiten 569-588, XP004313116 ISSN: 0168-583X**

EP 1 525 590 B1

**Beschreibung**

Die Erfindung betrifft einen abgeschirmten Raum für die Ionentherapie für Neutronen bis in den Energiebereich GeV.

In Deutschland und anderen europäischen Ländern werden zur Zeit medizinische Therapiebeschleunigeranlagen für hochenergetische Ionenstrahlung geplant [I]. Bei der Konzipierung von hochenergetischen Ionenbeschleunigern besteht die Problematik, dass Ionenbeschleuniger Sekundärstrahlung beim Abbremsprozess der Ionen in den Beschleunigerstrukturen, in biologischen oder anderen Targets produzieren. Die Hauptkomponente der Sekundärstrahlung ist Neutronenstrahlung. Diese Sekundärstrahlung ist ein unerwünschter Begleiteffekt. Der Primärstrahl hingegen wird beschleunigt bzw. transportiert.

Folgende Prozesse führen zu Erzeugung von Neutronen als Sekundärstrahlung:

- Strahlverluste durch Umladung;
- Strahlverluste durch Wechselwirkung mit dem Restgas durch unvollständiges Vakuum;
- während Auslenkungs- und Einlenkungsvorgängen, Extraktion und Injektion;
- bei der Abbremsung vom Ionenstrahl im Gewebe oder in sonstigen Materialien.

Die nicht zu vermeidende Sekundärstrahlung, die Neutronenstrahlung, muss abgeschirmt werden. Die als Quellstrahlung erzeugten Strahlenpegel sind erheblich und belaufen sich auf einem Level bis zu Sv/h. Die außerhalb der Abschirmung zu tolerierenden Strahlenpegel sind auf einem μSv/h-Niveau, je nach Definition der Bereiche außerhalb der Abschirmung, z.B. nach deutschen Strahlenschutzrecht Überwachungs- oder Kontrollbereiche. Somit muss die Neutronenstrahlung in ihrer Dosisleistung um ca. 6 Größenordnungen reduziert werden.

Die Realisierung einer Schwerionen-Therapieeinheit in klinischer Umgebung muss die Erfordernisse der Strahlenschutzverordnung berücksichtigen, d.h. angrenzende Bereiche an die Therapieräume sind als Überwachungsbereiche bzw. Bereiche zu definieren, die das 1-mSv-Limit pro Jahr bei vernachlässigbarer Emission von radioaktiven Stoffen einhält.

Konventionelle Therapieeinrichtungen, bei denen Patienten mit Röntgen- und Gammastrahlung bestrahlt werden, sind in Strahlenbunkern derart angeordnet, dass Betonwände die erzeugte Primärstrahlung wie die Streustrahlung so abschirmen, dass umgebende Bereiche praktisch keine erhöhten Strahlenpegel aufweisen.

Strahlentherapie wird bisher mit der Strahlung aus Gammastrahlungsquellen, wie etwa 137-Cs, 60-Co, oder mit in Elektronenbeschleunigern erzeugten Röntgenstrahlung durchgeführt. Der bauliche Strahlenschutz ist deshalb zur Abschirmung von Gamma- und Röntgenstrahlung. Für Elektronenbeschleuniger mit hohen Endpunktsenergien bis zu 50 MeV ist eine Abschirmung der im nuklearen Photoeffekt erzeugten Neutronenstrahlung notwendig. Maßgebende Regelwerke zum Design der Neutronenabschirmungen sind die DIN-Normen DIN 6847/Teil 2 [II], die Veröffentlichungen der NCRP und für Hochenergieteilchenbeschleuniger der Grundlagenforschung das Referenzwerk Landolt-Börnstein [III].

Die allgemeine Verfahrensweise zur Abschirmung von Neutronenstrahlung ist die Verwendung von wasserstoffhaltigen Substanzen, wie Wasser, Beton, wasserhaltige Materialien. Die für die Abschirmung von Röntgen- und Gammastrahlung verwendeten Stoffe, wie Blei oder Eisen, sind in geringerem Maße dazu geeignet, Neutronen zu schwächen oder zu absorbieren. Für direkte Neutronenstrahlung werden nach DIN 6847 Teil 2 [II] folgende Zehntelwertschichtdicken für den Bereich der medizinischen Bestrahlungsanlagen angegeben:

mSv-Limit pro Jahr, bei vernachlässigbarer Emission von

| Material | Wasser, Paraffin | Beton | Eisen, Blei |
|---|---|---|---|
| Zehntelwertdicke | 10 bis 15 cm | 16 bis 25 cm | 42 cm |

Aus Gründen der mangelnden Absorption und Schwächung von Neutronen der Energien bis 3 MeV wird in Metallen eine unzureichende Wirkung erzielt, so dass weitere wasserstoffhaltige Absorber im Anschluss an die Metallabschirmung verwendet werden müssen.

Die Abschirmwirkung einer Wand der Schichtdicke d und einer Mindestwandstärke ($d > d_0$) ist für einen Neutronenstrahl der Energie $E_n$:

$$\frac{H(d)}{H_0} = \frac{1}{r^2} \cdot \exp(-\frac{d}{\lambda(E_n, \vartheta)}) \; ,$$

mit der charakteristischen Schwächungskonstanten $\lambda(E_n)$, die von der Energie der Neutronenstrahlung und dem Winkel $\vartheta$ relativ zum einfallenden Strahl abhängt, dem Abstand $r$ zum Quellpunkt, der Abschirmdicke d und der Quellstärke $H_0$, die vom Primärstrahl und dem Target abhängt.

Die Abschirmwirkung ist für Kupfer im allgemeinen höher als für Beton mit Ausnahme für Neutronen mit Energien von 3 MeV oder kleiner. Die Wirkung von Abschirmungen sind jedoch derart, dass sämtliche Neutronenenergien beim Transport der Quellneutronen durch die Abschirmung auftreten können, so dass für alle Neutronenenergien wirksame Abschirmungen vorhanden sein müssen.

Die Strahlenschutzauslegungen der bisher errichteten Strahlentherapieanlagen konzentrierten sich hinsichtlich der Abschirmung der Neutronenkomponente auf den Energiebereich der Neutronen um 10 MeV. Hierbei ist Beton im allgemeinen allein ein hinreichend wirkender Strahlenschutz für alle Strahlenarten. Der Unterschied in der Abschirmwirkung ist für Metalle wie für Beton über einen weiten Energiebereich vergleichbar, nämlich zwischen 3 und 30 MeV.

Die jüngsten Strahlenschutzplanungen zum italienischen Ionentherapieprojekt TERA wurden von Agosteo et al. [IV] durchgeführt. In der Arbeit werden Kohlenstoffionen für die Planung zugrunde gelegt. Für die Auslegung der Strahlenschutzmaßnahmen werden Neutronenspektren verwendet, die auch hier zugrunde liegen. Agosteo entwickelte auf Basis der gemessenen Neutronenspektren und des Transports der Neutronen mit dem Strahlungstransportprogramm FLUKA [V]in vereinfachter Geometrie, z.B. sphärische Geometrie, ein Modell, das gestattet, die Schwächung der Neutronenstrahlung in vereinfachten Anordnungen abzuschätzen. Das Modell beschreibt im wesentlichen die Dosisleistung, verursacht durch Direktstrahlung. Zu erwartende erhöhte Anteile an der Dosisleistung durch Streustrahlung sind mit solchen Modellen nur schwer abzuschätzen.

Schwerionen-Therapieanlagen, die Tiefentherapie mit Kohlenstoffionen betreiben, benötigen beschleunigte Ionen mit Energien bis ca. 400 MeV pro Nukleon. Die beim Abbremsprozess der Ionen im Gewebe erzeugte Neutronenstrahlung weist Energien bis ca. 1000 MeV auf. Derart hochenergetische Neutronenstrahlung ist vor allem mit konventionellen Abschirmmaterialien schwer abzuschirmen. Die Schwächungslänge von Neutronen mit Energien größer als 100 MeV beträgt in Normalbeton der Dichte 2,3 g/cm$^3$ 45 bis 52 cm. Die Zehntelwertdicke beträgt ca. 100 cm.

Die physikalischen Parameter einer Ionen-Therapieanlage unterscheiden sich wesentlich von denen einer konventionellen Röntgenbestrahlungsanlage. So ist zwar der Primärstrahl, Protonen, Kohlenstoffionen, Sauerstoffionen, von der Erzeugung über die Beschleunigung bis zur Deponierung im Gewebe präzise geführt und wird nicht wie Röntgenstrahlung stark gestreut, aber es werden beim Abbremsprozess hochenergetische Neutronen erzeugt. So produziert ein Kohlenstoffion der Energie 400 MeV pro Nukleon beim Abbremsprozess ca. 5 Neutronen im Durchschnitt.

Ein weiterer grundlegender Unterschied zu konventionellen Röntgentherapieanlagen besteht in dem höheren räumlichen Aufwand und der räumlichen Aufteilung von der Strahlerzeugung bis hin zur Applikation des Strahls im Patienten. Dadurch ist allein der Abschirmaufwand für die Strahlführungen höher als bei konventionellen Anlagen. Weitere Konsequenzen sind ein erschwerter Zugang zu den Behandlungsräumen, da die Strahlführung selbst weite Bereiche um die Therapieeinheit belegt. Die bisherigen Abschirmkonzepte für Röntgenbestrahlungsanlagen nutzen hauptsächlich die Abschirmwirkung von Beton mit Schwächungslängen, die für MeV-Neutronenstrahlung anwendbar sind.

Strahlenschutzabschirmungen wurden bisher nur unter dem Aspekt von wasserstoffhaltigen Moderatoren zur Nutzung der elastischen Streuung von Neutronen an Protonen entwickelt, die mit zunehmender Neutronenenergie einen geringeren Wirkungsquerschnitt aufweisen. Konzepte zur Abschirmung von hochenergetischer Neutronenstrahlung müssen jedoch andere physikalische Prozesse berücksichtigen, wie Spallations- und Fragmentierungsreaktionen, die für hohe Energien konstante Wirkungsquerschnitte und konstante Wechselwirkungs-Wahrscheinlichkeiten, aufweisen. Im Vergleich zu Beton gibt es Materialien, die zu veränderten Schwächungslängen in den verschiedenen Materialien führen und kompakter ausfallen können als reine Betonabschirmungen.

Um die Gebäudestrukturen der Therapieeinheit effektiv dimensionieren zu können, ist von Interesse, kompakte Abschirmgeometrien zu planen. Dazu gehören:

- angrenzende Bereiche an die Therapieräume sind so zu gestalten, dass dauerhafter Personenaufenthalt vermieden werden kann;
- die Therapieräume in ihren Dimensionen so zu beschränken, dass nur kleine Bereiche abgeschirmt werden müssen;
- die Abschirmung selbst so effizient zu gestalten, dass sie wenig Volumen benötigt;
- Abschirnitüren aufgrund der zuletzt aufgeführten Maßnahmen in ihren Dimensionen so reduzieren zu können, dass sie auch ohne elektromotorische Kraft in akzeptablen Zeiten bewegt werden können.

Die der Erfindung zugrunde liegende Aufgabe besteht in der drittgenannten Maßnahme, nämlich die Abschirmung selbst so effizient zu gestalten, dass sie wenig Volumen benötigt.

Die Aufgabe wird in ihrem Kern durch den Anspruch 1 gelöst. Weitere, baulich zweckmäßige Maßnahmen sind in den Unteransprüchen 2 bis 10 beschrieben.

Die Lösung beruht auf dem Prinzip einer räumlich strukturierten Multi-Schicht-Komponentenabschirmung, der Kombinationsabschirmung für hochenergetische Neutronenstrahlung. Wesentlicher Bestandteil ist die Einführung einer Schicht, die zunächst die Wechselwirkung hochenergetischer Neutronen in Spallationsreaktionen mit schweren Atomkernen verursacht. Hierbei werden aus einem hochenergetischen Neutron mehrere niederenergetische Neutronen erzeugt. Diese Neutronen mit niederen Energien können dann mit konventionellen wasserstoffhaltigen Abschirmmaterialien absorbiert oder geschwächt werden. Die Kombinationsabschirmung wirkt in zwei Schritten:

- Auslösung von Spallations- und Fragmentierungsreaktionen in der ersten Schicht;
- Absorption der in ersten Schicht erzeugten Sekundärstrahlung. Wichtig bei der Kombinationsabschirmung ist eine Optimierung in verschiedenen Aspekten des Strahlenschutzes. In Spallationsreaktionen werden nicht nur Neutronen, sondern auch Kernfragmente darunter auch Radionuklide erzeugt, die ihrerseits wiederum eine Quelle möglicher Strahlung sind.

Die Wahl der schweren Targetkerne gibt vor, welches Spektrum an Radionukliden produziert werden kann. Die Auswahl des schweren Targetkerns optimiert somit die Umsetzung von hochenergetischen in niederenergetische Neutronen und der Erzeugung von Beta- und Gammastrahlen emittierender Radionuklide. Die Verwendung von schweren Spallations-Neutronenkonvertern, Blei, Wismut etc., verursacht ein entsprechend großes Spektrum an erzeugten teilweise auch längerlebigen Radionukliden. Leichtere Spallationsneutronen-Konverter haben eine geringere Effizienz für die Spallation, jedoch sind die Möglichkeiten und die Wirkungsquerschnitte zur Produktion von Radionukliden geringer, da entsprechend der Nuklidkarte weniger Radionuklide erzeugt werden können. Dies ist ein Vorteil im Sinne einer Strahlenschutzbetrachtung bezüglich der Exposition durch Beta- und Gammastrahlung nach Abschalten des Beschleunigers.

Die Kombinationsabschirmung lässt sich mit Maßnahmen der räumlichen Strukturierung folgendermaßen zusammenfassen:

i. Einführung von Kombinationsabschirmungen, z.B. Metall mit 0,5 bis 1 m Schichtdicke und anschließend wasserstoffhaltige Schicht mit 1,5 bis 2 oder 3 m Schichtdicke, bei denen in der ersten Schicht hochenergetische Neutronen Spallationsreaktionen verursachen und Neutronen vorwiegend geringerer Energie erzeugt werden, die in der zweiten und weiteren Schichten geschwächt werden, z.B. 0,5 m Fe und 1,5 m Beton.

ii. Einführung von Querstreben von der Decke senkrecht abwärts in Richtung Boden, beispielsweise aus Beton.

iii. Einführung von querstehenden Wänden in Kombinationsabschirmung, mehrere Kombinationsabschirmungen hintereinander.

vi. Strahlenschutztür aus wasserstoffhaltigen Substanzen, wie Polyethylen, Wasser, Paraffin etc.

v. Eine weitere Maßnahme ist ein - Rückstreuraum für Neutronen mit Wänden in Kombinationsabschirmung und seitlichem Ausgang ins Labyrinth.

vi. Strahlstoppverstärkung für die Stelle in der der Primärstrahl vernichtet wird.

Eine zusätzliche Maßnahmen ist:

- ein mobile Abdeckung, z.B. aus Metall wie Pb, für den Bereich in dem der Primärstrahl vernichtet wird,

um die erzeugten gammastrahlenden Nuklide abzuschirmen. Hier kann die Abdeckung während des Strahlbetriebs in einer Abschirmung versenkt werden um eine Aufaktivierung zu vermeiden und nach Abschaltung des Primärstrahls kann dann diese bewegliche Abschirmung an die abzuschirmende Stelle gefahren werden.

Weitere mögliche Maßnahme sind:

- die mobile Abschirmungen im Bestrahlungsraum, sog. Stellwände.

Die o.g. Maßnahmen müssen kombiniert werden, um den geforderten Abschirmeffekt zu erzielen. Die Verwendung der Kombinationsabschirmung ist Bestandteil einer optimierten Strahlenschutzanordnung.

Die Einzelmaßnahmen sind aus der Literatur bekannt oder finden in der Praxis der Beschleuniger der Grundlagenforschung Anwendung. In ihrer Kombination zur Optimierung einer Abschirmwirkung sind sie neu. In ihrer Kombination wirken sie derart, dass eine Reduzierung der Dosisleistung der erzeugten Sekundärstrahlung um ca. 6 Größenordnungen von der Strahlenquelle zum Eingang hin erzielt werden kann. Wesentlicher Aspekt ist sowohl die Reduzierung des Anteils an direkter Strahlung von der Quelle als auch gleichermaßen des Anteils an Streustrahlung. Bisherige Abschirmungen für Neutronenstrahlung nutzen lediglich über die Abbremsung der einfallenden Neutronen, wasserstoffhaltiger Moderator, die vorherrschende Neutroneneinfangreaktion in der Abschirmung aus.

Die hier vorgeschlagenen Maßnahmen erzeugen zunächst einmal über Spallationsreaktionen der einfallenden Strahlung Neutronen mit durchschnittlich kleinerer Energie als die einfallenden Neutronen. Diese erzeugten Neutronen werden dann wie bisher moderiert und über Neutroneinfangreaktionen in der Abschirmung absorbiert. Weiterhin werden neue geometrische Elemente für die Abschirmstrategie im Labyrinthdesign eingeführt, wie z. B. die Rückstreusackgasse zur Reduzierung der Streustrahlung.

Die o.g. Strategien bewirken je nach Applikation der aufgeführten Maßnahmen unter folgenden Aspekten eine optimierte Abschirmung der erzeugten Neutronen- und Photonenstrahlung:

- Reduzierung der Strahlenpegel, verursacht durch Direktstrahlung ungestreute Strahlung;
- Reduzierung der Streustrahlung;
- Optimierung der Ausnutzung des räumlichen Aufwands der Abschirmeinheiten;
- die Produktion von Radionukliden durch Sekundärstrahlung, deren Minimierung und die Reduzierung der Strahlenexposition durch die erzeugten gamma- und betastrahlenden Nuklide;
- Kombination der ersten drei Maßnahmen derart, dass z.B. eine Zugangsabschirmtür überflüssig wird.

Die wesentlichen Vorteile der Erfindung liegen in der Realisierung des baulichen Strahlenschutzes. Diese sind:

- Optimierung der Abschirmung hinsichtlich Material und Geometrie;
- Zusammenfassung der optimierten Maßnahmen zur Erfüllung der Vorgaben aus der Strahlenschutzverordnung im Hinblick auf Anlagen zur Krebsbehandlung mittels Schwerionentherapie mit Protonen, Kohlenstoffionen und andere Ionen;
- die vorgeschlagenen Maßnahmen sind, selbst für den ungünstigen Fall der Eingangsrichtung zum Behandlungsplatz des Patienten auf der Strahlachse eine adäquate Abschirmung zu gewährleisten, geeignet;
- effizientere Abschwächung der Direkt- und Streustrahlung für hochenergetische Neutronestrahlung;
- kompakte Abschirmmaßnahme für einen Zweimodusbetrieb mit Patientenbestrahlung und Qualitätssicherung mit Strahlvernichtung in der Abschirmwand;
- Berücksichtigung der Erzeugung von radioaktiven Stoffen während der Bestrahlung und der Abschirmung dieser Stoffe.

Die derzeitige Prototypplanung einer Schwerionentherapieanlage an einer radiologischen Universitätsklinik ist für Kohlenstoffionenstrahl und Protonenstrahl ausgelegt. Die Planung wurde vom TÜV-Süddeutschland in Hinblick auf die Abschirmanordnung und -wirkung geprüft und in ihrer prinzipiellen Konzeption bestätigt.

Die Erfindung wird anhand der Skizze des Horizontalbestrahlungsplatzes der geplanten Schwerionen-Therapieeinrichtung für diese Universitätsklinik näher beschrieben. Dazu wird die Zeichnung mit zwei Figuren herangezogen. Diese zeigen im einzelnen:

Figur 1 die Skizze des Horizontalbestrahlungsplatzes der geplanten Schwerionen-Therapieeinrichtung an der Universitätsklinik in Heidelberg und

Figur 2 Modellierung der Geometrie P für die Horizontalbestrahlungsplätze durch das Strahlungstransportprogramm FLUKA zur Bestimmung der Umgebungsäquivalentdosis H*(10) für Neutronen.

Der Betrieb der Schwerionentherapieanlage mit jeweils Protonen, Kohlenstoff-Ionen, Neon-Ionen und schwerere Ionen als Primärstrahl, ist mit der Produktion von Neutronenstrahlung verbunden. Grund sind die geschilderten kernphysikalischen Fragmentierungsprozesse des Ions oder des Targetkerns während des Abbremsvorgangs des Ions in Materie. Zum Beispiel werden pro Kohlenstoffion mit der Energie 400 MeV pro Nukleon im Durchschnitt 5 Neutronen freigesetzt. Neben der mit minimalen Verlusten behafteten Strahlführung vom Synchrotron zum Patienten ist vor allem der Bereich der Patientenbehandlung ein Ort erhöhter Neutronendosisleistung.

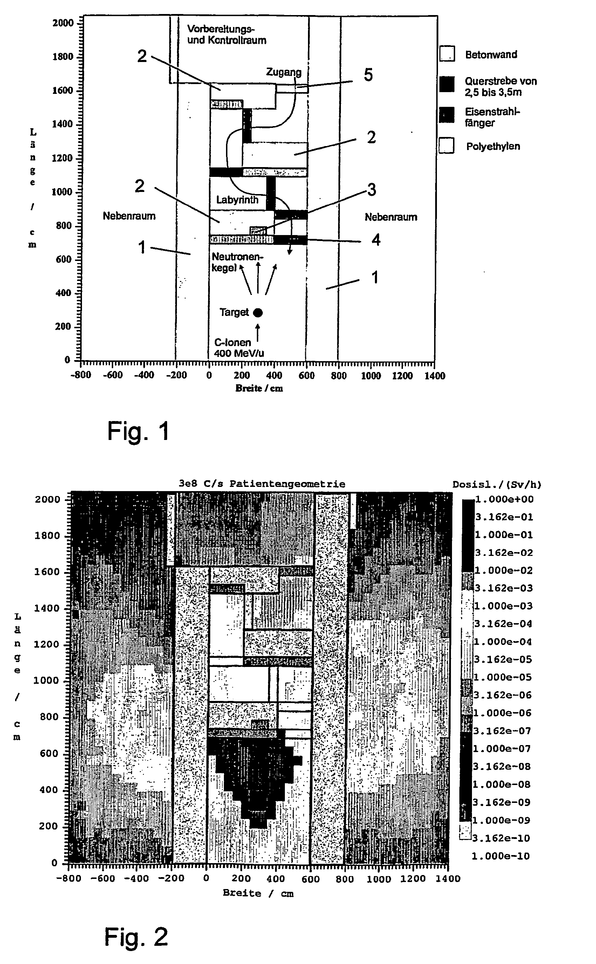

Durch die Anordnung derartiger Therapieanlagen liegt der Zugang zum Behandlungsbereich in manchen Fällen vis à vis des Primärstrahls auf der Strahllinie. Die räumliche Abstrahlung der Neutronen ist derart, dass der Hauptanteil der Neutronen in Vorwärtsrichtung des Ionenstrahls gestreut wird. Dies erzwingt die Konstruktion des Zugangsbereichs mit Maßnahmen derart, dass die Strahlung bis zum Eingangsbereich des Therapieortes genügend geschwächt wird (siehe Figur 1 und 2).

Die Bestimmung der Wirkung der Abschirmungsanordnungen zu einem Bestrahlungsplatz ist am genauesten mit Strahlungstransportprogranmnen durchzuführen. Im Ausführungsbeispiel sind Ergebnisse von Monte-Carlo-Strahlungstransportrechnungen, die auf gemessenen Neutronenspektren basieren, zusammengefasst.

Die Abschätzung der Strahlenpegel wurde für die Horizontalbestrahlungsplätze unter Anwendung des Strahlungstransportprogramms FLUKA berechnet, basierend auf gemessenen Neutronenspektren. Als Strahl wird ein Kohlenstoffionenstrahl mit einer Energie von 400 MeV pro Nukleon berücksichtigt. Als Target diente für die Messungen ein Graphitblock mit 100 x 100 x 200 mm³ bzw. ein Kupfertarget mit 100 x 100 x 50 mm³. Die Neutronenspektren wurden unter den Winkeln 0°, 7,5°, 15°, 30°, 60° und 90° gemessen.

Es gibt grundsätzlich zwei Bestrahlungsmodi für derartige TherapieAnlagen:

- Bestrahlung von Patienten mit einer vollen Primärstrahldeponierung im Patienten;
- Bestrahlung zur Verifizierung von Bestrahlungsplänen und zur Qualitätssicherung, bei denen der Strahl auch in einem Strahlfänger deponiert werden kann.

Für die Berechnungen wurden die mit einem Kohlenstofftarget gemessenen Spektren als repräsentativ für eine Patientenbestrahlung bzw. die mit einem Kupfertarget gemessenen als stellvertretend für die Bestrahlungen zur Qualitätssicherung betrachtet.

Für die FLUKA-Simulationsrechnungen des Labyrinths werden die zwei Geometrien folgendermaßen behandelt:

- Patientengeometrie, P;
- Geometrie Q (Qualitätssicherung) für Kontrollmessungen.

Im ersten Fall wird der Strahl im Target ca. 3,5 m vor der ersten Wand, im letzteren Fall wird der überwiegende Teil des Strahls direkt in der ersten Wand des Labyrinths vernichtet (siehe Fig. 1 und 2). Die produzierten Neutronen werden durch das Labyrinth in Richtung des Eingangsbereichs transportiert und die Dosisleistungen entlang des Labyrinths ermittelt. Für die Intensität des Ionenstrahls erwartet man, gemittelt über ein 10-Minuten-Zeitintervall im Betrieb, maximale Werte von $10^7$ Ionen pro Sekunde. Der theoretisch maximale Wert liegt bei $3 \cdot 10^8$ Ionen pro Sekunde. Für Quellneutronen, die rückwärts gestreut werden, werden die unter 90° gemessenen Neutronenspektren verwendet.

Zur Optimierung der Abschirmwirkung werden folgende Maßnahmen eingeführt:

- Labyrinthtechnik zur Reduzierung der Streustrahlung;
- neben-konventionellen Betonabschirmungen, Verwendung von Kombinationsabschirmungen;
- Einführung von Querstreben von der Decke bis auf 2,5 m Höhe an den Durchgängen der Zwischenwände;
- lokale Verstärkung der Eisenabschirmung in der ersten Wand von 0, 5 m auf 1,0 m für die Dump-Funktion auf einer Fläche von 1 m² für die Qualitätssicherung;
- Verwendung einer Eisenabschirmung auch in der dritten Quer-Wand (0,5 m);
- Verwendung einer Polyethylentür im Eingangsbereich (0,5 m Dicke);
- Verwendung von Eisenabschirmungen im Deckenbereich über dem Target (0,5 m Eisen).

Die Ermittlung der Dosisleistungen konzentrieren sich auf den Eingangsbereich zum Labyrinth, wo Aufenthaltsbereiche für das Personal sind, und auf die benachbarten Räume. Es sind die Dosisleistungswerte berechnet für einen Primärstrahl maximaler Intensität mit $3 \cdot 10^8$ Ionen pro Sekunde (siehe Fig. 2).

Die u.s. Tabelle 1 gibt einen Überblick der durchgeführten Berechnungen. Geometrie P bedeutet Patientengeometrie, Geometrie Q ist die Bestrahlung zur Qualitätssicherung. Geometrie D bedeutet Dachgeometrie, hier mit einer Eisenverstärkung von 0,5 m Eisen als Kombinationsabschirmung statt einer 2 m dicken Betonabschirmung über der Target-Stelle berücksichtigt.

Die Besonderheit der hier geplanten Anordnung - Vorwärtsstreuung des Neutronenkegels in den Zugang - wird bisher in der Literatur für diese Art von Bestrahlungsanlagen nicht berücksichtigt.

Tabelle 1: Überblick der mit FLUKA berechneten Geometrien unter Verwendung der verschiedenen Strahlparameter und Geometrien.

| Strah[Ionen/sec] | Dosisleistung | Geometrie | Abbildung |
|---|---|---|---|
| $3 \cdot 10^8$ | Neutronen | P | 4 |
| $3 \cdot 10^8$ | Photonen | P | 5 |
| $1 \cdot 10^7$ | Neutronen | P | 6 |
| $3 \cdot 10^8$ | Neutronen | Q | 7 |
| $1 \cdot 10^7$ | Neutronen | Q | 8 |

(fortgesetzt)

| Strah[Ionen/sec] | Dosisleistung | Geometrie | Abbildung |
|---|---|---|---|
| $3* \cdot 10^8$ | Neutronen | D | 9 |
| $1* \cdot 10^7$ | Neutronen | D | 10 |

**Bezugszeichenliste:**

1. Abschirmung
2. Abschirmung
3. Bereich
4. Abschirmung
5. Abschirmtür
6. Bereich

**Literatur:**

[I] .
J. Debus; Proposal for a dedicated Ion Beam Facility for Cancer Therapy. DKFZ, GSI and FZR Report 1998

[II].
DIN 6847 / Teil 2 (Strahlenschutzregeln für die Errichtung von medizinischen Elektronenbeschleunigeranlagen, Kapitel 8.7, Bemessung der Abschirmung von Neutronenstrahlung), März 1990.

[III].
A. Fasso, K. Goebel, M. Höfert, J. Ranft, G. Stevenson in Landolt-Börnstein, Gruppe 1: Kern- und Teilchenphysik; Band 11: Abschirmung gegen hochenergetische Strahlung, Herausgeber H. Schopper, Springer-Verlag, Berlin, 1990.

[IV].
S. Agosteo; Radiation Protection at Medical Accelerators; Radiation Protection Dosimetrie, Vol. 96No. 4, 393-406 (2001).

[V] .
A. Fasso, A. Ferrari, J. Ranft, P.R. Sala: New developments in FLUKA, modelling hadronic and EM interactions Proc. 3rd Workshop on Simulating Accelerator Radiation Environments, KEK, Tsukuba (Japan) 7-9 May 1997. Ed. H. Hirayama, KEK Proceedings 97-5 (1997), p. 32-43.

**Patentansprüche**

1. Abgeschirmter Raum für die Ionentherapie mit Abschirmwirkung für Neutronen bis in den Energiebereich GeV, bestehend aus:

   einem Therapieraum,
   dessen Hauptachse durch die Richtung des Therapiestrahls vorgegeben wird, der bis auf eine Seite allseitig mit einer Abschirmung (1) versehen ist und
   einem abschirmenden labyrinthartigen Zugang an der nicht abgeschirmten Seite des Therapieraums,

   wobei der labyrinthartige Zugang aus mindestens zwei bezüglich der Strahlachse in der Länge versetzten Abschirmungen (2) besteht, die von entgegengesetzten seiten in den Raum ragen und jeweils mehr als die Hälfte der lichten Weite des Zugangs abdecken,
   wobei die Abschirmungen (2) als Multi-Schicht-Komponenten abschirmung ausgestaltet sind, so dass sie geeignet sind Spallationsvorgänge der einfallenden Neutronen auszulösen und die produzierten Neutronen zu moderieren.

2. Abgeschirmter Raum nach Anspruch 1, **dadurch gekennzeichnet, dass** die Abschirmungen (2) auf der dem Therapieraum zugewandten Seite eine Metallschicht aufweisen.

3. Abgeschirmter-Raum nach Anspruch 1 oder 2, **dadurch gekennzeichnet, dass** die eine der beiden Abschirmungen (2), die dem Therapieraum zugewandt ist, im Bereich (3), in dem der Primärstrahl auftrifft, stärker ausgeführt ist.

4. Abgeschirmter Raum nach einem der Ansprüche 1 bis 3, **dadurch gekennzeichnet, dass** im labyrinthartigen Zugang zwischen den beiden Abschirmungen (2) zusätzliche wasserstoffhaltige Abschirmungen (4) angebracht sind.

5. Abgeschirmter Raum nach einem der Ansprüche 1 bis 4,
**dadurch gekennzeichnet, dass** im labyrinthartigen Zugang mindestens eine weitere Abschirmung (2) angeordnet ist, wobei die Abschirmungen abwechselnd links und rechts anstoßen.

6. Abgeschirmter Raum nach einem der Ansprüche 1 bis 5,
**dadurch gekennzeichnet, dass** der labyrinthartigen Zugang an seiner dem Therapiebereich abgewandten Seite eine wasserstoffhaltige Abschirmtür (5) enthält.

7. Abgeschirmter Raum nach einem der Ansprüche 1 bis 6,
**dadurch gekennzeichnet, dass** der Bereich (3), in dem der Primärstrahl auftrifft, eine konkave Oberfläche aufweist.

8. Abgeschirmter Raum nach einem der Ansprüche 1 bis 7,
**dadurch gekennzeichnet, dass** Teile der Abschirmung (1) so ausgestaltet sind, dass sie geeignet sind Spallationsvorgänge der einfallenden Neutronen auszulösen und die produzierten Neutronen moderiert werden können.

9. Abgeschirmter Raum nach einem der Ansprüche 1 bis 8,
**dadurch gekennzeichnet, dass** der Bereich (3) durch ein zusätzliches bewegliches Abschirmelement abgeschirmt werden kann.

10. Abgeschirmter Raum nach einem der Ansprüche 1 bis 9,
**dadurch gekennzeichnet, dass** das freie Ende von mindestens einem der Abschirmungen (2) T oder L- förmig ausgestaltet ist.

## Claims

1. Shielded room for ion therapy with a neutron shielding effect up to the GeV energy range, consisting of:

   a therapy room,
   the main axis of which is given by the direction of the therapy beam and all sides, except for one side, of which are provided with a shielding (1),
   and a shielding labyrinth-like access on the unshielded side of the therapy room, with this labyrinth-like access consisting of at least two shieldings (2) that are offset in length relative to the beam axis, extend into the room from opposite sides, and cover more than half of the clearance of the access each, with these shieldings (2) being designed as multi-layer component shieldings suited for the initiation of spallation processes of incident neutrons and moderation of the neutrons produced.

2. Shielded room according to Claim 1, **characterized by** the shieldings (2) on the side facing the therapy room being provided with a metal coating.

3. Shielded room according to Claim 1 or 2, **characterized by** one of both shieldings (3), whichever is closer to the therapy room, being thicker in the range (3) of incidence of the primary beam.

4. Shielded room according to one of Claims 1 through 3, **characterized by** additional hydrogen-containing shieldings (4) being installed in the labyrinth-like access between both shieldings (2).

5. Shielded room according to one of Claims 1 through 4, **characterized by** at least another shielding (2) being arranged in the labyrinth-like access area, with all shieldings contacting the left and right wall in an alternating manner.

6.  Shielded room according to one of Claims 1 through 5, **characterized by** the labyrinth-like access, on the side turned away from the therapy area, being provided with a hydrogen-containing shielding door (5).

7.  Shielded room according to one of Claims 1 through 6, **characterized by** the area (3) of incidence of the primary beam having a concave surface.

8.  Shielded room according to one of Claims 1 through 7, **characterized by** the parts of the shielding (1) being designed, such that they are suited for initiating spallation processes of the incident neutrons and for moderating the neutrons produced.

9.  Shielded room according to one of Claims 1 through 8, **characterized by** the fact that the area (3) may be shielded by an additional movable shielding element.

10. Shielded room according to one of Claims 1 through 9, **characterized by** the free end of at least one of the shieldings (2) being designed in an T- or L-shape.


**Revendications**

1.  Chambre blindée pour la thérapie ionique avec effet de blindage pour des neutrons jusqu'à la plage d'énergie GeV, comprenant :

    une chambre de thérapie,
    dont l'axe principal est prédéfini par la direction du rayon de thérapie, qui à l'exception d'un côté est munie de tous les côtés d'un blindage (1), et
    un accès de blindage en forme de labyrinthe du côté non blindé de la chambre de thérapie,
    l'accès en forme de labyrinthe comprenant au moins deux blindages (2) décalés longitudinalement par rapport à l'axe de rayon, qui font saillie dans la chambre depuis des côtés opposés et recouvrent chacun plus que la moitié de la largeur libre de l'accès, les blindages (2) étant des blindages à plusieurs couches de composants, de façon à pouvoir déclencher des processus de spallation des neutrons incidents et modérer les neutrons produits.

2.  Chambre blindée selon la revendication 1,
    **caractérisée en ce que**
    les blindages (2) situés du côté tourné vers la chambre de thérapie présentent une couche métallique.

3.  Chambre blindée selon la revendication 1 ou 2,
    **caractérisée en ce que**
    l'un des deux blindages (2) tourné vers la chambre de thérapie est plus épais dans la zone (3) dans laquelle arrive le rayonnement primaire.

4.  Chambre blindée selon l'une quelconque des revendications 1 à 3,
    **caractérisée par**
    des blindages (4) supplémentaires à base d'hydrogène disposés dans l'accès en forme de labyrinthe, entre les deux blindages (2).

5.  Chambre blindée selon l'une quelconque des revendications 1 à 4,
    **caractérisée par**
    au moins un autre blindage (2), dans l'accès en forme de labyrinthe, les blindages étant aboutés en alternance à gauche et à droite.

6.  Chambre blindée selon l'une quelconque des revendications 1 à 5,
    **caractérisée en ce que**
    du côté détourné de la zone de thérapie l'accès en forme de labyrinthe comporte une porte de blindage (5) à base d'hydrogène.

7.  Chambre blindée selon l'une quelconque des revendications 1 à 6,
    **caractérisée en ce que**
    la zone (3) dans laquelle arrive le rayonnement primaire présente une surface concave.

8.  Chambre blindée selon l'une quelconque des revendications 1 à 7,
    **caractérisée en ce que**
    des parties du blindage (1) sont aptes à déclencher des processus de spallation des neutrons incidents et à modérer les neutrons produits.

9.  Chambre blindée selon l'une quelconque des revendications 1 à 8,
    **caractérisée en ce que**
    la zone (3) peut être blindée par un élément de blindage mobile supplémentaire.

10. Chambre blindée selon l'une quelconque des revendications 1 à 9,
    **caractérisée en ce que**
    l'extrémité libre d'au moins l'un des blindages (2) est en forme de T ou de L.

Fig. 1

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **J. DEBUS.** Proposal for a dedicated Ion Beam Facility for Cancer Therapy. *DKFZ, GSI and FZR Report,* 1998
- DIN 6847 / Teil 2. *Strahlenschutzregeln für die Errichtung von medizinischen Elektronenbeschleunigeranlagen, Kapitel 8.7, Bemessung der Abschirmung von Neutronenstrahlung,* Marz 1990
- Landolt-Börnstein, Gruppe 1: Kern- und Teilchenphysik. **A. FASSO ; K. GOEBEL ; M. HÖFERT ; J. RANFT ; G. STEVENSON.** Abschirmung gegen hochenergetische Strahlung. Springer-Verlag, 1990, vol. 11
- **S. AGOSTEO.** Radiation Protection at Medical Accelerators. *Radiation Protection Dosimetrie,* 2001, vol. 96 (4), 393-406
- **A. FASSO ; A. FERRARI ; J. RANFT ; P.R. SALA.** *New developments in FLUKA, modelling hadronic and EM interactions Proc. 3rd Workshop on Simulating Accelerator Radiation Environments,* 07. Mai 1997, vol. 97-5, 32-43